(19) Europäisches Patentamt
European Patent Office
Office européen des brevets

(11) Publication number: **0 100 983**
**A1**

# (12) EUROPEAN PATENT APPLICATION

(21) Application number: 83107538.7

(22) Date of filing: 31.07.83

(51) Int. Cl.³: C 07 J 9/00
C 07 C 93/00, A 61 K 31/575

(30) Priority: 17.08.82 IT 2287382

(43) Date of publication of application:
22.02.84 Bulletin 84/8

(84) Designated Contracting States:
AT BE CH DE FR GB LI LU NL SE

(71) Applicant: PROTER S.p.A.
38 Via Lambro
I-20090 Opera (Milan)(IT)

(72) Inventor: Dall'Asta, Leone
via D. Chiesa
Pavia(IT)

(72) Inventor: Coppi, Germano
Via G. Morandi, 5
I-20094 Buccinasco (MI)(IT)

(72) Inventor: Scevola, Mario Ercole
via Tuberose, 4
Milano(IT)

(74) Representative: Gervasi, Gemma et al,
Studio Brevetti e Marchi NOTARBARTOLO & GERVASI
33, Viale Bianca Maria
I-20122 Milano(IT)

## (54) Steroid compounds with choleretic activity, a process for their preparation, and therapeutic compounds which contain them as active principle.

(57) New salts of steroid acids with trimebutine are prepared, of formula:

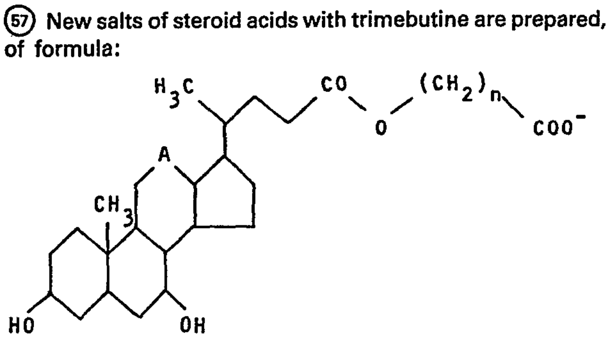

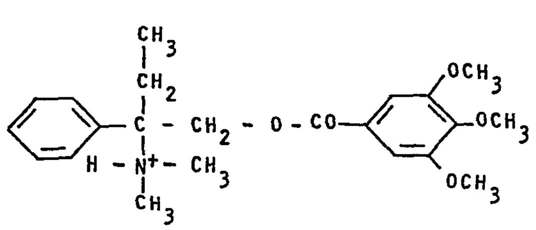

In the acid, A can be =CO, =CH–OH or $=CH_2$, and n is between 1 and 4.

Furthermore, the OH group in position 3 is in α and that in position 7 can be in α - or in β -; if A is CHOH, the OH group in position 12 is in α.

The new compounds have a choleretic action comparable with that of ursodeoxycholic acid in terms of intensity, but is considerably more prolonged in time, and is associated with an antispastic action which is approximately double that of trimebutine.

EP 0 100 983 A1

STEROID COMPOUNDS WITH CHOLERETIC ACTIVITY, A PROCESS FOR THEIR PREPARATION, AND THERAPEUTIC COMPOUNDS WHICH CONTAIN THEM AS ACTIVE PRINCIPLE

This invention relates to new compounds of general formula:

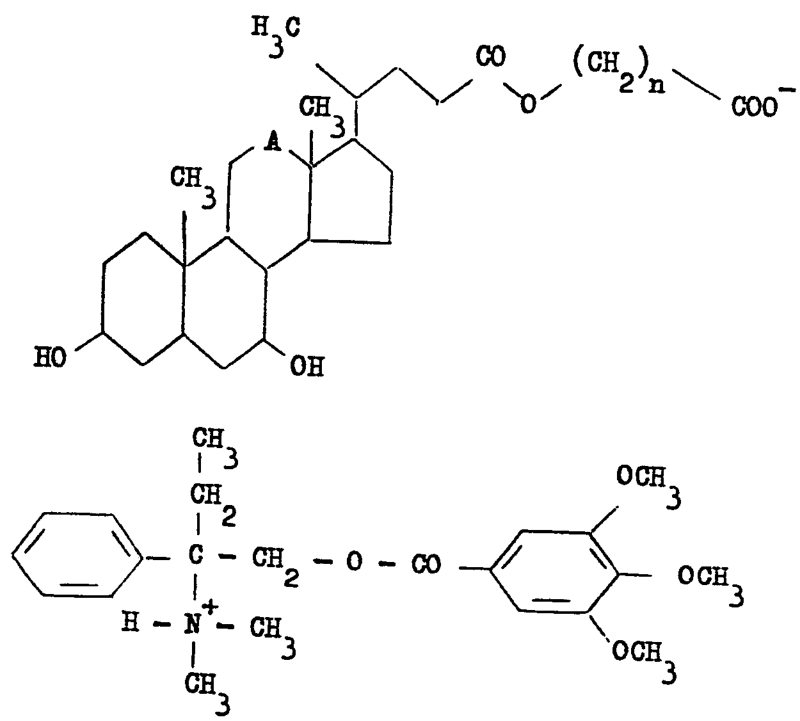

(I)

where A can be -CO-, -CHOH- or $-CH_2-$, and n is between 1 and 4. In addition, the OH group in position 3 is in α - and that in position 7 can be in α - or in β -; if A is -CHOH-, the OH group in position 12 is in α -.

The invention also relates to processes for preparing the compounds of formula I.

It also relates to pharmaceutical compositions containing the compounds of formula I as active principle.

It is known that acids of formula II:

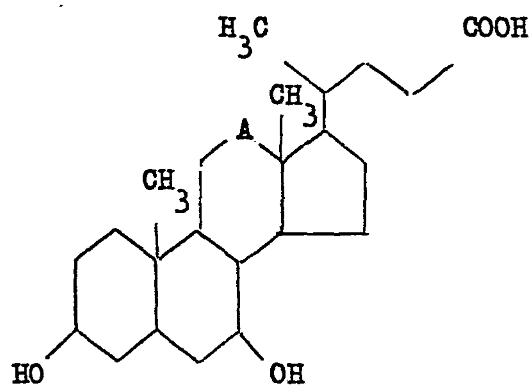

(II)

where A has the aforesaid meaning, and in which the OH groups are in positions α - or β - as heretofore specified, possess interesting pharmacological activity.

These compounds all have the property of increasing the bile flow and the bile salt content of the bile itself. Under these conditions, much more cholesterol is dissolved, and this prevents its precipitation in the form of calculi.

However, these compounds have the drawback of being poorly soluble and of not giving rise to the formation of soluble salts. In addition, their choleretic action becomes depleted after a short time.

It is also common knowledge that trimebutine (2-phenyl-2-dimethyl-amino-n.butylester of 3,4,5-trimethoxybenzoic acid) is used in human therapy in the treatment of altered kinesis of the gastro-intestinal tract.

We have now synthesised the new products of formula (I) and have found that they are active both in biliary dyskinesia and in the altered kinesis of the gastro-intestinal tract, and that both these activities are considerably more powerful in intensity and

duration than those of known cholic acids and trimebutine respectively.

Moreover, the new products are soluble and can therefore be administered parenterally.

The acids which form part of the molecule of formula (I) are synthesised from compounds of formula:

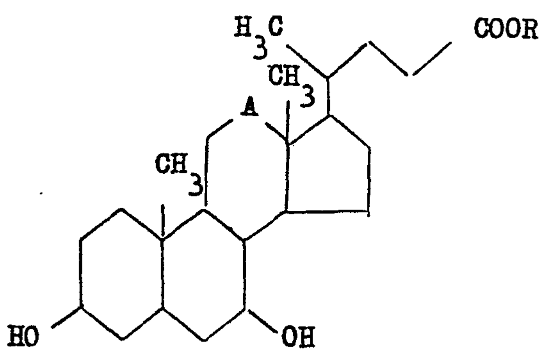

(IIa)

in which R is H, an alkaline metal or a reactive group, by reaction with carboxylic acid derivatives of formula

$$R_1 - (CH_2)_n - COOR \qquad (III)$$

in which $R_1$ is OH or halogen, and $R_2$ is H or a benzyl group.

More specifically, the acids of formula I are synthesised by the two following processes:

a - reacting acid II in the form of one of its reactive derivatives in a suitable solvent with a solution of a hydroxyacid of formula:

$$HO - (CH_2)_n - COOH$$

where n has the aforesaid meaning.

b - reacting a salt of acid II or the acid itself with benzyl alcohol haloesters of formula:

$$X - (CH_2)_n - COO - CH_2 - C_6H_5$$

where X is a hydrogen atom, and n has the aforesaid meaning.

The condensation product obtained, which has the formula:

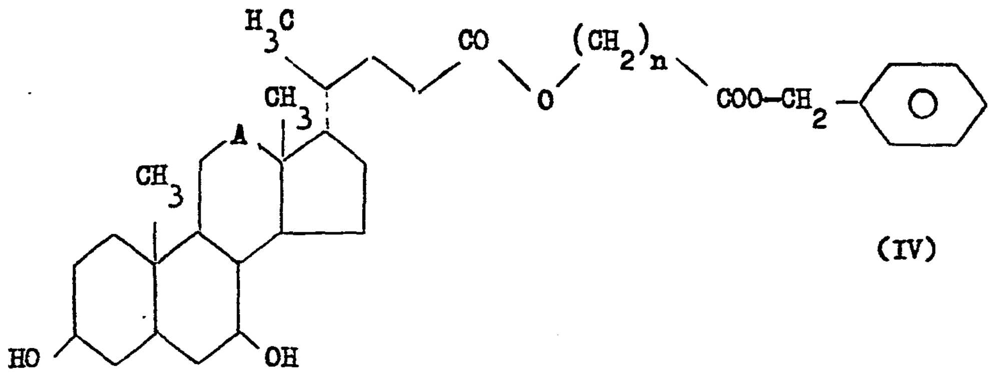

(IV)

is subjected to hydrogenolysis to give the product of formula I.

According to process a), the reactive derivative of acid II is prepared by reacting a salt of said acid deriving from an organic base, for example n-tributylamine, with an alkylchloroformate, for example ethyl chloroformate, in stoichiometric quantities in an anhydrous solvent, for example dioxane, at a temperature of between $-10^{o}$ and $+30^{o}C$, preferably between $+5^{o}$ and $+10^{o}C$, for a time of between 10 minutes and 8 hours, and preferably between 15 minutes and 1 hour.

An aqueous solution of a salt of the hydroxyacid, for example its sodium salt, is added to the mixed anhydride solution of acid II obtained, at a temperature of between $0^{o}$ and $+40^{o}C$, and preferably between $+5^{o}$ and $+10^{o}C$.

The reaction is complete in a time of between 30 minutes and 48 hours. In the case of very slow reactions it is advantageous to add additional quantities of the aqueous solution of the hydroxyacid salt to the extent of 10-20% of the initial quantity.

The product of formula I is isolated by traditional methods.

In process b), the condensation of acid II with haloesters of formula IV, for example benzyl bromoacetate, takes place in a polar solvent, for example N,N-dimethylformamide, in the presence of an HX acid acceptor such as potassium carbonate. Alternatively, the alkaline salt of the acid is firstly prepared, and then

reacted with the acid halide.

The reaction takes place at a temperature of between 25° and 150°C, and preferably between 50° and 60°C. On termination of the reaction, which occurs after between 30 minutes and 12 hours of heating, and preferably between 4 and 5 hours, the product of formula IV is isolated by normal separation and purification methods.

The product IV is subjected to hydrogenolysis in polar solvents, for example acetic acid, in the presence of a catalyst, such as 5% palladium on carbon. When hydrogen absorption is complete, the catalyst is removed by filtration, and the solution evaporated under reduced pressure to isolate the acid by conventional methods.

The acid obtained in this manner is salified with trimebutine by reaction in stoichiometric proportions in a suitable organic solvent at a temperature of between 20° and 60°C.

The salt (I) is separated by evaporating the solvent followed by recrystallisation.

The following examples illustrate the invention but without limiting it.

EXAMPLE 1

Preparation of 3α,7β-dihydroxy-6β-cholan-24-oyl-acetic acid (I, OH-3α, OH-7β, A = $-CH_2-$, n = 1)

15 g (0.0382 moles) of 3α,7β-dihydroxy-6β-cholan-24-oic acid (ursodeoxycholic acid) are added to 132 ml of dioxane, followed by 9.1 ml (0.0382 moles) of n.tributylamine. The mixture is heated under agitation at 30°-32°C until complete solubilisation is attained.

After cooling to +10°C, 3.64 ml (0.0382 moles) of ethyl chloroformate

are added, and the mixture then kept at between +8° and +10°C for 15 minutes.

19.7 ml of a 2.13 M aqueous solution of sodium glycolate are then added.

During this addition, the temperature is kept at between +8° and +10°C, after which the mixture is cooled to +5°C and kept under these conditions for three hours.

Chromatographic analysis (TLC - Merck 60F254 silica gel plates - eluent: chloroform/acetic acid/water 85:15:5 (v/v/v), shows the presence of a small quantity of the starting acid, and a further 3.58 ml of the 2.13 M aqueous solution of sodium glycolate are added.

After 15 hours at +5°C, the mixture is evaporated under reduced pressure at 40°C to give a semi-solid residue; this is dispersed in 250 ml of water.

The aqueous phase (pH 5.5) is removed by decanting from the separated solid. The residue is dissolved in 600 ml of 10% aqueous $NaHCO_3$ solution. After decoloration by charcoal and filtration, the filtrate is acidified to pH 2 by adding 6N HCl.

The precipitate obtained is filtered, and washed with water until the $Cl^-$ reaction disappears in the filtrate.

The precipitate is dried under vacuum at 40°C to give 11.3 g (65%) of white product having a M.P. of 94°-96° (dec.) and $[\alpha]_D^{20}$ of +49.5° (c = 2, ethanol).

Elementary analysis:
calculated: C 69.30%; H 9.39%
found: C 68.84%; H 9.33%

IR and NMR spectra conform to the assigned structure.

HPLC analysis (refractive index) shows the presence of less than 0.2% of ursodeoxycholic acid.

EXAMPLE 2

Preparation of 3α,7β-dihydroxy-6β-cholan-24-oyl acetic acid (I, OH-3α, OH-7β, A = $-CH_2-$, n = 1)

a) Benzyl ester of 3α,7β-dihydroxy-6β-cholan-24-oyl acetic acid (IV, OH-3α, OH-7β, A = $-CH_2-$, n = 1):

19.7 g (0.0503 moles) of 3α,7β-dihydroxy-6β-cholan-24-oic acid are added to 490 ml of N,N-dimethylformamide, followed by 3.5 g (0.0253 moles) of finely pulverised potassium carbonate. The mixture is kept under agitation at 50°C for 1 hour, after which 12.8 g (0.0558 moles) of benzyl bromoacetate are added and the mixture kept at 50°C for 4 hours.

It is then evaporated under vacuum at 40°-50°C to obtain a semi-solid residue, which is dissolved in 400 ml of chloroform.

The organic phase is separated, washed firstly with 600 ml of a 5% aqueous solution of $NaHCO_3$, then twice with 300 ml of water.

After drying with anhydrous sodium sulphate, it is filtered, and the filtrate evaporated under reduced pressure to give 26.6 g (98%) of colourless oil.

The product purity is verified by TLC (Merck F254 silica gel plates - eluent: chloroform/acetic acid/water 85:15:0.5 (v/v/v)).

Elementary analysis:
calculated: C 73.30%; H 8.95%
found: C 73.49%; H 8.88%

The IR and NMR spectra conform to the assigned structure.

0100983

b) 3α,7β-dihydroxy-6β-cholan-24-oyl acetic acid (I, OH-3α, OH-7β, A = $-CH_2-$, n = 1):

28 g (0.0518 moles) of IV (OH-3α, OH-7β, A = $-CH_2-$, n = 1) are dissolved in 415 ml of glacial acetic acid, and 4.15 g of 5% palladium on carbon moist with 50% water are added.

The suspension under strong agitation is subjected to hydrogenation in a Parr apparatus at atmospheric pressure and ambient temperature. Absorption of the theoretical volume of $H_2$ (1115 ml) is complete in about 1 hour.

The catalyst is filtered off and the filtrate evaporated under reduced pressure at 40°C to give a dense colourless oil.

The oil is taken up in 800 ml of a 10% aqueous $NaHCO_3$ solution. After decoloration with charcoal and filtration, the filtrate is acidified to pH 2 by adding 6N HCl. The white precipitate is filtered off, and washed with distilled water until the $Cl^-$ reaction disappears in the filtrate.

It is dried under vacuum at 40°C.

Yield: 13.5 g (58%) of white product with a M.P. of 94°-95.5°C (dec.), and $[\alpha]_D^{20}$ of +49.95° (c = 2, ethanol).

HPLC analysis shows 0.12% of ursodeoxycholic acid.

The IR and NMR spectra conform to the assigned structure.

EXAMPLE 3

Preparation of the 2-dimethylamino-2-phenylbutyl 3,4,5-trimethoxybenzoate salt of 3α,7β-dihydroxy-6β-cholan-24-oyl acetic acid.

13.52 g (0.03 moles) of the acid obtained in Example 1 or 2 are dissolved in 1200 ml of anhydrous ethyl alcohol by heating to

0100983

40°C. 11.63 g (0.03 moles) of 2-dimethylamino-2-phenylbutyl 3,4,5-trimethoxybenzoate (trimebutine) are added to the solution obtained.

The clear solution is concentrated under reduced pressure to a small volume, and 2 volumes of n-heptane are added.

The crystalline product is filtered off and washed with n-heptane on the filter.

It is dried under vacuum at 40°C.

Yield: 17.8 g (71%) of white product of M.P. 108°-112°C (dec.) and $[\alpha]_D^{20}$ of + 24.4° (c = 2. ethanol).

Elementary analysis:
calculated: C 68.79%; H 8.54%; N 1.67%
found: C 68.77%; H 8.49%; N 1.658%

The IR and NMR spectra conform to the assigned structure.

As stated initially, the new salts possess considerable choleretic activity which is prolonged in time.

By way of example, details are given of the activity of 3α,7β-dihydroxy-6β-cholan-24-oyl acetic acid (A) and of its trimebutine salt (B) compared with ursodeoxycholic acid (C), using the method of D'Armour and coll. (J. Pharmacol. Exp. Theor. 133, 400 (1961)).

This method consists essentially of cannulating the choledocus of the treated animals and collecting the bile before and after treatment with the drugs.

24 male albino rats of Wistar stock were used having an average weight of 400 g and divided into 4 batches of 6 animals each.

In the method used, after the surgical cannulation, the bile was allowed to flow out for about 30-45 minutes in order to regulate the flow, after which the basic bile was collected for 60 minutes.

After this time, the drug was administered into the subpyloric duodenal tract at a dose of 0.5 mM/kg, and the bile was collected in suitable graduated test tubes, the samples of each animal being split into periods of exactly 60 minutes, for a time of 3 hours after treatment. The following were then determined for each animal:

a) the quantity of bile which had flown out after each hour, and
b) the dry residue obtained after drying each sample under vacuum.

The tested compounds were solubilised with stoichiometric quantities of M NaOH, to obtain solutions or suspensions at pH 10.

Physiological solution (S.F.) adjusted to pH 10 by M NaOH was used for the control group.

The data obtained in the described experiments by administering equimolar doses of the products A and B and of the comparison drug C are shown on the accompanying graphs.

More specifically, the graph of Figure 1 shows the percentage variation in the bile flow with time (test a) and the graph of Figure 2 shows the percentage variation in the bile dry residue with time (test b), and compared with the controls (S.F.).

The graph of Figure 1 clearly shows that for equal doses the compounds A and B have a maximum intensity action on the bile flow which is practically equivalent to that of the comparison drug. Their activity is slightly less after 1 hour but is greater after 2 hours. With salt B, this activity is high even after 3 hours.

The graph of Figure 2 shows that the dry residual content of the

bile (indicative of the bile salt content) attains maximum values after treatment with compounds A and B which are practically equal to that obtained after treatment with ursodeoxycholic acid.

However, this test also shows that the action of the new salt lasts about double (2 hours) the action of ursodeoxycholic acid (1 hour).

Compound B was also used in "in vitro" antispastic activity tests on the guinea pig ileum, using acetylcholine bromide as antagonist in comparison with trimebutine hydrochloride.

The new product demonstrated an antispastic activity approximately double that of the comparison compound.

Acute toxicity was also tested by oral administration of the acid A and the salt B in the rat in comparison with ursodeoxycholic acid. 60 Wistar rats of the two sexes were used having an average weight of 200 g, divided randomly into three batches of 10 males and 10 females and treated orally by gastric probe with the following products:

batch 1: ursodeoxycholic acid (C) - 15% suspension in a 10% solution of gum arabic; 20 mg/kg equivalent to 3 g/kg of product;

batch 2: product (A) - 15% suspension in a 10% solution of gum arabic: 20 ml/kg equivalent to 3 g/kg of product;

batch 3: product (B) - 15% suspension in a 10% solution of gum arabic: 20 ml/kg equivalent to 3 g/kg of product.

The animals, kept under observation for two weeks, registered no case of death or other toxicity symptom.

The two compounds A and B are very well tolerated when administered orally in a single dose even of massive proportions, and their behaviour is comparable with that registered in the rat after administering ursodeoxycholic acid.

Their acute toxicity by oral administration in the rat ($LD_{50}$) is obviously greater than 3 g/kg.

Concluding, it can be stated that the new salt B possesses choleretic action similar to that of ursodeoxycholic acid but considerably more prolonged in time, associated with antispastic action approximately double that of trimebutine, and with absolute absence of toxicity.

The new salts are therefore useful in human therapy in the treatment of dyskinesia, biliary dyspepsia, cholicystopathies, and in the normalisation of kinesis in the intestinal gastro-biliar tract.

Some examples of pharmaceutical preparations of choleretic activity using compound B according to the present invention are given hereinafter for illustrative purposes only.

From the examples given, it can be seen that the human therapeutic doses vary preferably, but not exclusively, between 100 and 300 mg two or three times per day. The new products are administered preferably orally in the form of capsules or tablets.

Capsules with different dosages:

| | | | | |
|---|---|---|---|---|
| 1) | <u>Capsule</u>: | each capsule contains: | | |
| | | - Active principle - | Compound (B) | 100 mg |
| | | - Excipients - | Starch | 18 mg |
| | | | Mg stearate | 6 mg |
| | | | Precipitated silica | 6 mg |
| 2) | <u>Capsule</u>: | each capsule contains: | | |
| | | - Active principle - | Compound (B) | 200 mg |
| | | - Excipients - | Starch | 36 mg |
| | | | Mg stearate | 12 mg |
| | | | Precipitated silica | 12 mg |

Tablets with different dosages:

3) <u>Tablet</u>: each tablet contains:

| | | | |
|---|---|---|---|
| - Active principle - | Compound (B) | 100 mg |
| - Excipients - | Lactose | 150 mg |
| | Starch | 25 mg |
| | Gum arabic | 5 mg |
| | Talc | 16 mg |
| | Mg stearate | 4 mg |

4) Tablet: each tablet contains:

| | | |
|---|---|---|
| - Active principle - | Compound (B) | 200 mg |
| - Excipients - | Lactose | 150 mg |
| | Starch | 25 mg |
| | Gum arabic | 5 mg |
| | Talc | 16 mg |
| | Mg stearate | 4 mg |

5) Tablet: each tablet contains:

| | | |
|---|---|---|
| - Active principle - | Compound (B) | 300 mg |
| - Excipients - | Lactose | 200 mg |
| | Starch | 25 mg |
| | Gum arabic | 5 mg |
| | Talc | 16 mg |
| | Mg stearate | 4 mg |

Moreover, the new salts are water-soluble so that they can be easily administered parenterally in considerably lower doses.

PATENT CLAIMS

1. Compounds of formula:

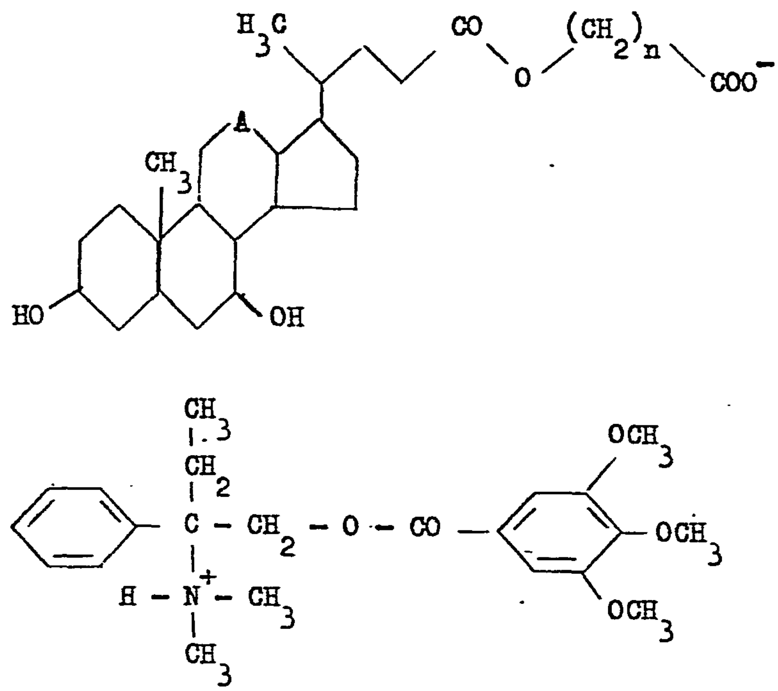

(I)

in which A is =CO, =CH-OH or $=CH_2$, and n is between 1 and 4.

2. , A process for preparing compounds of formula:

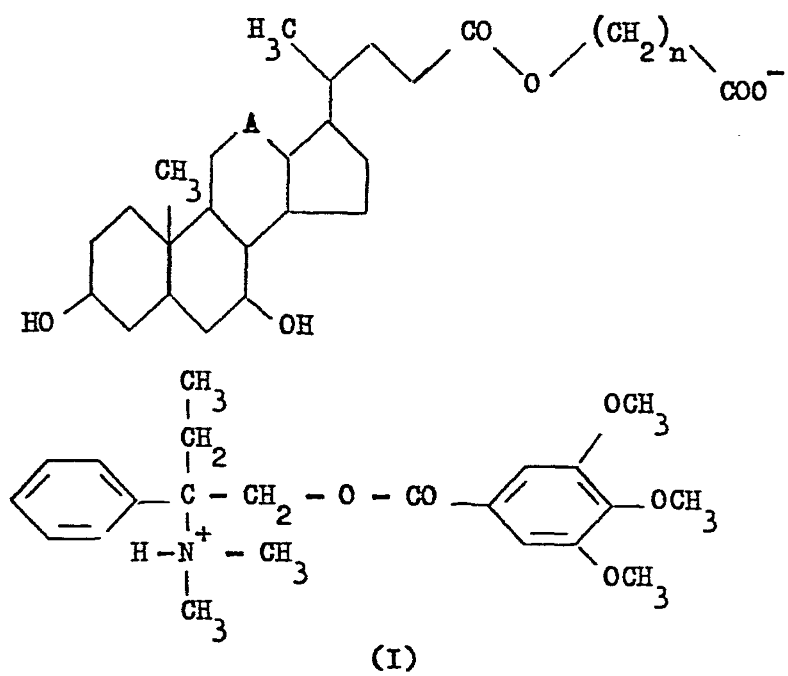

(I)

in which A is =CO, =CH-OH or $=CH_2$, and n is between 1 and 4 characterised by reacting a compound of formula:

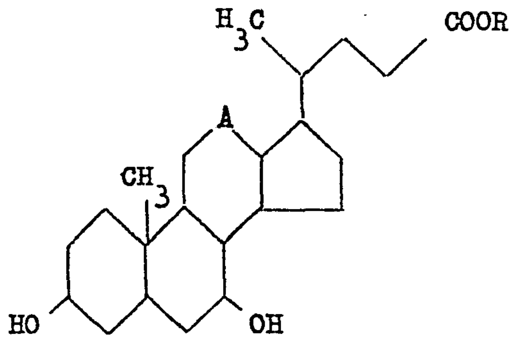

(IIa)

in which R is an alkaline metal or a reactive group, with a compound of formula:

$$R_1 - (CH_2)_n - COOR_2 \qquad (III)$$

in which $R_1$ is OH or halogen, and $R_2$ is H, an alkaline metal or a benzyl group, and the acid obtained is salified with the stoichiometrically equivalent quantity of trimebutine.

3. A process as claimed in claim 2, wherein a solution of the compound of formula (IIa), in which R is a reactive group, is reacted with the aqueous solution of a compound of formula (III), in which $R_1$ is OH and $R_2$ is H or an alkaline metal, at a temperature of between $0^o$ and $40^o$ C, and the acid obtained is salified with the stoichiometrically equivalent quantity of trimebutine in an organic solvent solution at a temperature of between $20^o$ and $60^oC$.

4. A process as claimed in claim 2, wherein a solution of the compound of formula (IIa), in which R is H or an alkaline metal, is reacted with a solution of the compound of formula (III), in which $R_1$ is halogen and $R_2$ is benzyl, possibly in the presence of a hydrohalogen acid acceptor, at a temperature of between $25^o$ and $150^oC$, the benzyl derivative thus obtained is subjected to hydrogenolysis in a polar solvent, and the acid obtained is salified with the stoichiometrically equivalent quantity of trimebutine.

5. Therapeutic compositions of choleretic and antispastic activity, characterised by comprising as active principle at least one compound of formula (I) in mixture with therapeutically acceptable excipients and diluents.

PATENT CLAIMS FOR AUSTRIA

1. A process for preparing compounds of formula:

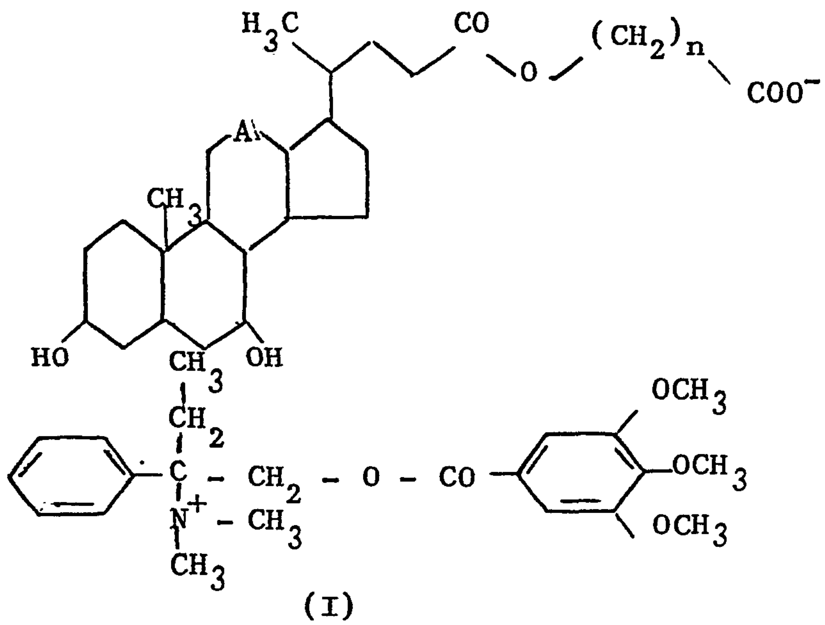

(I)

in which A is =CO, =CO-OH or $=CH_2$, and n is between 1 and 4 characterised by reacting a compound of formula:

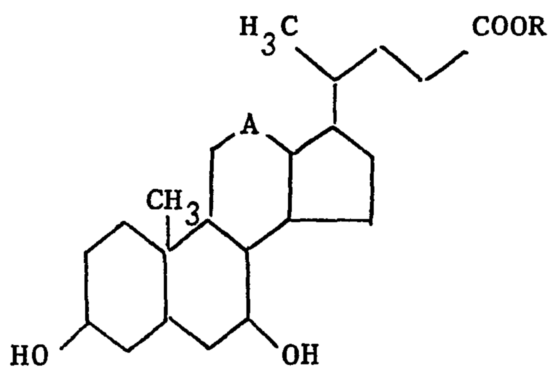

(IIa)

in which R is an alkaline metal or a reactive group, with a compound of formula:

$$R_1 - (CH_2)_n - COOR_2 \qquad \text{(IIIa)}$$

in which $R_1$ is OH or halogen, and $R_2$ is H, an alkaline metal or a benzyl group, and the acid obtained is salified with the stoichiometrically equivalent quantity of trimebutine.

2. A process as claimed in claim 1, wherein a solution of the compound of formula (IIa), in which R is a

reactive group, is reacted with the aqueous solution of a compound of formula (III), in which $R_1$ is OH and $R_2$ is H or an alkaline metal, at a temperature of between 0° and 40° C, and the acid obtained is salified with the stoichiometrically equivalent quantity of trimebutine in a organic solvent solution at a temperature of between 20° and 60° C.

3. A process as claimed in claim 1, wherein a solution of the compound of formula (IIa), in which R is H or an alkaline metal, is reacted with a solution of the compound of formula (III), in which $R_1$ is halogen and $R_2$ is benzyl, possibly in the presence of a hydrohalogen acid acceptor, at a temperature of between 25° and 150°C, the benzyl derivative thus obtained is subjected to hydrogenolysis in a polar solvent, and the acid obtained is salified with the stoichiometrically equivalent quantity of trimebutine.

FIG 1

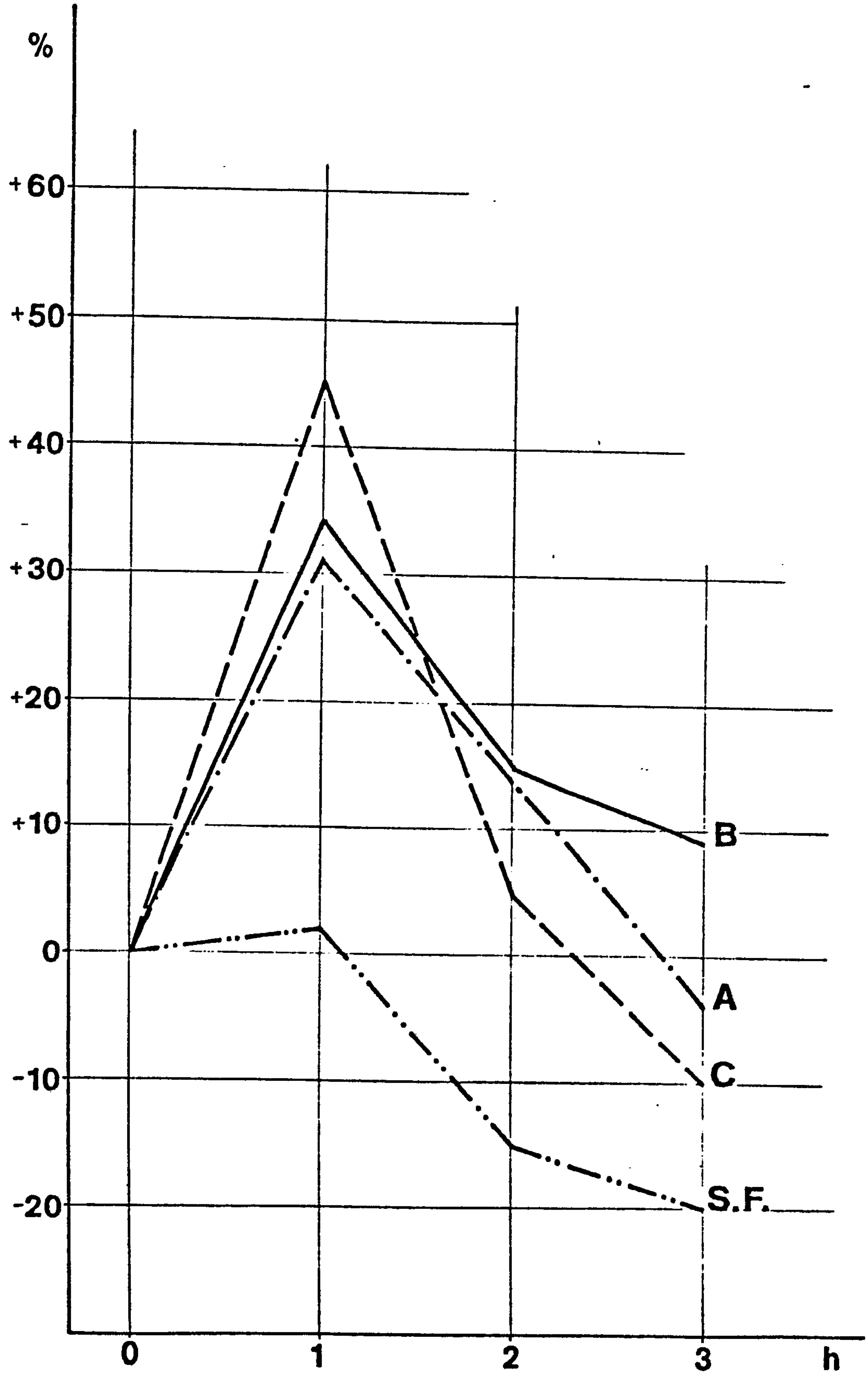

2/2

0100983

FIG 2

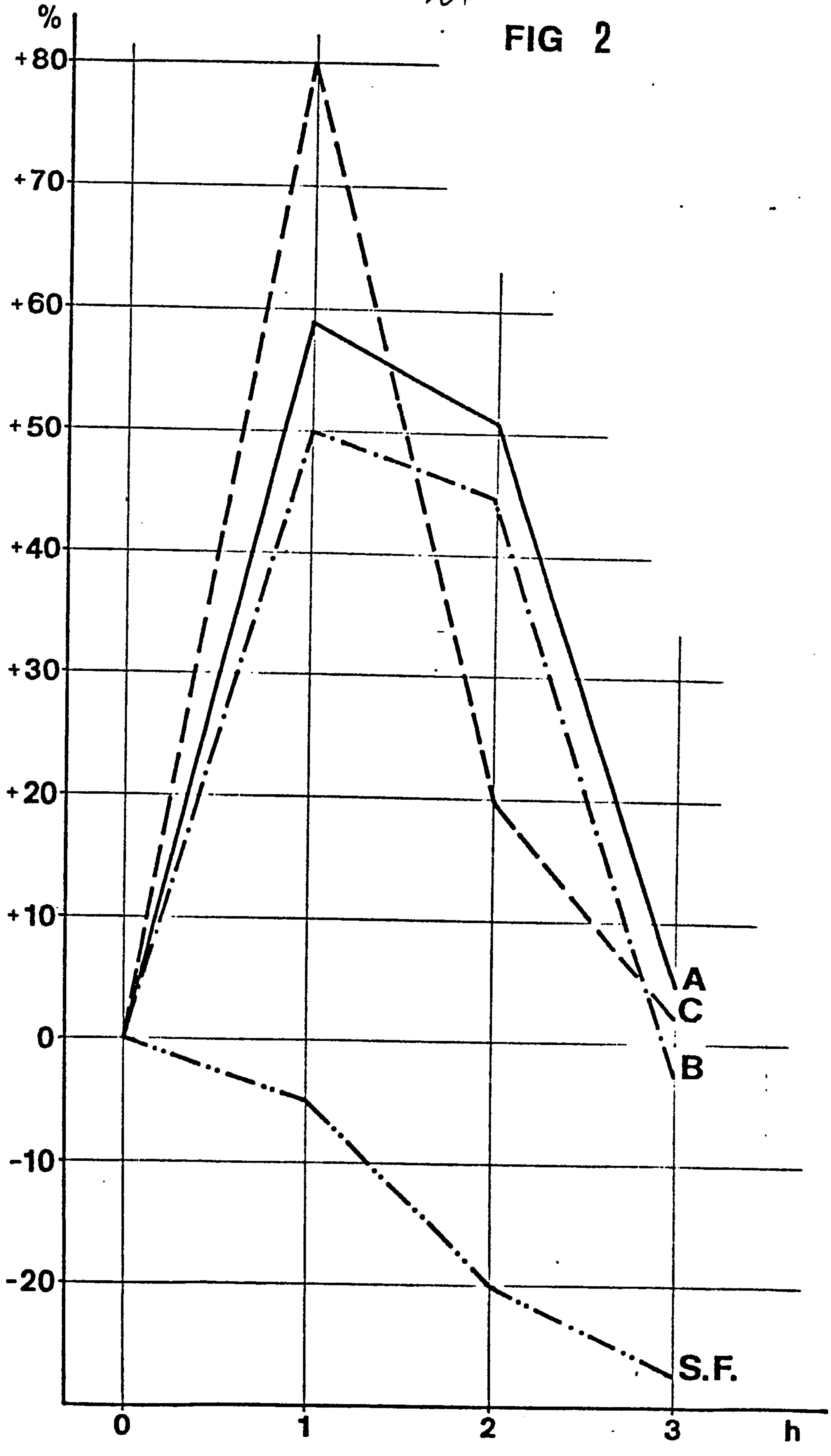

0100983

European Patent Office

# EUROPEAN SEARCH REPORT

Application number

EP 83 10 7538

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | FR-M- 2 369 (LABORATOIRES JOUVEINAL)<br>* Abstract *<br>--- | 1,5 | C 07 J 9/00<br>C 07 C 93/00<br>A 61 K 31/575 |
| A | US-A-3 910 888 (JOSEF OLAF WIDAUER)<br>* Claims *<br>--- | 1,5 | |
| P | EP-A-0 065 666 (ERREGIERRE S.p.A.)<br>* Claims *<br>----- | 1-5 | |
| | | | **TECHNICAL FIELDS SEARCHED (Int. Cl. 3)** |
| | | | C 07 J 9/00<br>C 07 C 93/00 |

The present search report has been drawn up for all claims

| Place of search | Date of completion of the search | Examiner |
|---|---|---|
| THE HAGUE | 22-11-1983 | HENRY J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82